# EUROPÄISCHE PATENTSCHRIFT

(11) **EP 2 026 750 B1**
(45) Veröffentlichungstag und Bekanntmachung des Hinweises auf die Patenterteilung: **16.06.2010**
(21) Anmeldenummer: 07726245.9
(22) Anmeldetag: 29.01.2007
(51) Int. Cl.: A61K 8/34, A61K 8/37, A61Q 5/06, A61Q 11/00, A61Q 13/00, C12F 5/00

(54) **VERGÄLLUNGSMITTEL FÜR ETHANOL SOWIE DIESEN ENTHALTENDES KOSMETISCHES ODER MUNDHYGIENISCHES PRODUKT**
DENATURING AGENT FOR ETHANOL AND ITS COSMETIC OR ORAL HYGIENIC PRODUCT
DÉNATURANT DE L'ÉTHANOL ET PRODUIT COSMÉTIQUE OU D'HYGIÈNE BUCCO-DENTAIRE CONTENANT CELUI-CI

(30) Priorität: 28.04.2006 DE 102006020332
(43) Veröffentlichungstag der Anmeldung: 25.02.2009
(73) Patentinhaber: Symrise GmbH & Co. KG, 37603 Holzminden (DE)
(72) Erfinder: MACHINEK, Arnold, 37603 Holzminden (DE)
(74) Vertreter: Sendrowski, Heiko
(86) Internationale Anmeldenummer: PCT/EP2007/050822
(87) Internationale Veröffentlichungsnummer: WO 2007/124963

(56) Entgegenhaltungen:
- EP-A- 0 876 811
- JP-A- 4 283 240
- US-A- 5 885 553
- DATABASE WPI Week 200002 Derwent Publications Ltd., London, GB; AN 2000-021680 XP002443159 & RU 2 115 705 C1 (KHAIREDINOV YU E) 20. Juli 1998 (1998-07-20)
- DATABASE WPI Week 198413 Derwent Publications Ltd., London, GB; AN 1984-078370 XP002443160 & JP 59 029618 A (HASEGAWA CO LTD) 16. Februar 1984 (1984-02-16)

## Beschreibung

Die vorliegende Erfindung betrifft ein Vergällungsmittel für Ethanol (efindungsgemäße Zusammensetzung), Ethanol mit einem Gehalt dieses Vergällungsmittels (erfindungsgemäße Mischung) und kosmetische sowie mundhygienische Produkte mit einem Gehalt dieses vergällten Ethanols.

Ethylalkohol, der nicht für den Verzehr (z.B. als Bestandteil eines Lebens- und Genussmittels) bestimmt ist, wie z.B. Ethylalkohol für die Herstellung von Kosmetika, ist aus steuerlichen Gründen durch Zusatz von sogenannten Vergällungsmitteln für den Verzehr bzw. Genuss für den Menschen unbrauchbar zu machen. Unter Vergällung versteht man das Denaturieren des Ethanols, wodurch er für den menschlichen Genuss (als Trinkalkohol) unbrauchbar gemacht wird und somit die für unvergälltes Ethanol geltende Steuer nicht entrichtet werden muss. Zu diesem Zweck können verschiedene Vergällungsmittel eingesetzt werden. Das weltweit am häufigsten eingesetzte Vergällungsmittel ist Diethylphthalat.

Die Wahl des Vergällungsmittels ist dabei vom Einsatzzweck des Ethanols abhängig. Nach Artikel 17 der Verordnung (EWG) Nr. 2454/93 muss die Mischung aus zu vergällendem Erzeugnis und Vergällungsmittel homogen sein. Die Bestandteile der Mischung dürfen in wirtschaftlich sinnvoller Weise nicht mehr getrennt werden können. Diethylphthalat ist für Kosmetika das gebräuchlichste Vergällungsmittel, aber auch andere Mittel wie Thymol, Isopropanol oder tert.-Butylalkohol werden eingesetzt (siehe auch Branntweinsteuerverordnung, BGBI I 1994, 104).

In den U.S.A. ist die Vergällung von Alkohol durch 27 CFR Part 21 geregelt [Title 27 (Alcohol, Tobacco and Firearms), Part 21 (Formulas for denatured alcohol and rum), dort speziell 27 CFR § 21.64 - 21.133).

Die bekannten Vergällungsmittel weisen regelmäßig (teils erhebliche) Nachteile auf, welche die Produktqualität mindern. Diethylphthalat beispielsweise verursacht in Aerosolformulierungen Niesreiz. Ferner beeinflusst Diethylphthalat die geruchlichen bzw. geschmacklichen Eigenschaften von parfümierten bzw. aromatisierten Produkten in nicht kontrollierbarer Weise. Andere Vergällungsmittel wie z.B. Isopropanol oder Moschus Keton beeinflussen den Geruchscharakter von parfümierten Produkten und sind teilweise farblich instabil. Vergällungsmittel müssen bzw. sollen nicht zum Verzehr in größeren Mengen geeignet sein, sollten dabei jedoch, insbesondere bei der Verwendung in kosmetischen bzw. mundhygienischen Produkten, nicht nennenswert gesundheitsschädlich sein, d.h. beispielsweise im Falle eines unbeabsichtigten Verschluckens nicht zu Gesundheitsproblemen führen.

EP 0 876 811 (entsprechend US 5,968,535) beschreibt ein Vergällungsmittel für Ethanol umfassend a) Benzylsalicylat, b) (i) 4.6.6.7.8.8.-Hexamethyl-1.3.4.6.7.8.-hexahydrocyclopenta[g]benzopyran oder (ii) Oxacyclohexadec-12- und/oder -13-en-2-on, c) hydrierte Wurzelöl-C₁-C₄-alkylester, d) 2-Pentylphenylpropanol, e) Benzylbenzoat, f) Dipropylenglykol und g) Triethylcitrat. Zwar eignen sich solche Vergällungsmittel für den Einsatz in topischen Kosmetikprodukten, für die Verwendung in Mundhygieneprodukten weisen diese Vergällungsmittel aber einen zu dominierenden Eigengeschmack auf, der nur schwer mit anderen Riech- und Aromastoffen kombinierbar ist bzw. geruchlich bzw. geschmacklich für den Konsumenten nicht akzeptabel ist.

DE 2746108 A1offenbart (Seite 17, Beispiele 3 und 4) Lotionen zur Behandlung von Akne, enthaltend u.a.

| | |
|---|---|
| 25 Gew.-% | Ethanol absolut |
| 38,4 Gew% | Propylenglycol |
| 0,2 Gew.-% | p-Methylbenzoat |
| 10 Gew.-% | Ethyllactat. |

US 2006/0068066 A eine flüssige Zusammensetzung, die bei Untersuchungen des Sorptionsverhaltens von Aromastoffen angewendet wird. Die flüssige Zusammensetzung enthält u.a. ein Alkohol als Lösungsmittel, z.B. Ethanol und eine Esterkomponente ausgewählt aus u.a. Milchsäureestem wie cis-3-hexenyllactat, trans-3-hexenyllactat oder Benzoesäureestern wie Methylbenzoat, Ethylbenzoat, Propylbenzoat und Butylbenzoat.

JP 04283240 A offenbart ein Lösungsmittel für Epoxidharze, enthaltend mindestens eine Komponente ausgewählt u.a. Ethyllactat, Butyllactat, Amyllactat, Methylbenzoat, Ethylbenzoat, Butylbenzoat, Diethylmalonat.

US 5 612 303 A offenbart eine Lösungsmittelzusammensetzung zum Auflösen von Polymeren. Die Zusammensetzung enthält mindestens eine Verbindung ausgewählt aus u.a. Methyllactat, Ethyllactat, Butyllactat, Methylbenzoat, Ethylbenzoat, Propylbenzoat, Diethylmalonat und Propylenglycol.

Die Veröffentlichungen von P. Schreier et al in Sci.Fd. Agric. 29(1978) 728-736 und Z. Lebensm. Unters. Forsch. 167 (19798) 16-22 betreffen Untersuchungen der Zusammensetzung der Aromastoffe bei der Herstellung von Apfelbranntwein (apple brandy). Der Apfelbranntwein (apple brandy) hat einen Alkoholgehalt von 52 Vol.-%. Unter den nachgewiesenen Aromastoffen sind u.a. Ethyllactat, Isobutyllactat, Isopentyllactat, Ethylbenzoat und Diethylmalonat.

Aufgabe der vorliegenden Erfindung war es daher, ein (alternatives) Vergällungsmittel zu finden, das einen mit Diethylphthalat vergleichbaren Vergällungseffekt erlaubt ohne die geschilderten Nachteile aufzuweisen. Das gesuchte Vergällungsmidel sollte lediglich einen schwachen Eigengeruch bzw. Eigengeschmack aufweisen, so dass es mit Riech- und Aromastoffen gut kombiniert und in kosmetische bzw. mundhygienische Produkte eingearbeitet werden kann, d.h. es soll insbesondere in kosmetischen bzw. mundhygienischen Produkten universell einsetzbar sein. Ferner sollte das Vergällungsmittel bei Zugabe von Wasser zu einem entsprechenden vergällten Ethanol bzw. beim Vermischen von Wasser und einem entsprechenden vergällten Ethanol nicht zu Trübungen oder Entmischung der Phasen (d.h. nicht zur Ausbildung eines Ölfilms oder von "Fettaugen") führen.

Die gestellte Aufgabe wird gelöst durch eine Zusammensetzung umfassend oder bestehend aus:
A) 20 bis 80 Gew.-% an einem oder mehreren Milchsäure-C₂-C₆-estern, vorzugsweise Ethyllactat, n-Butyllactat, iso-Amyllactat und/oder n-Amyllactat, und
B) 1 bis 20 Gew.-% an einem oder mehreren Benzoesäure-C₁-C₄-estern, vorzugweise Methylbenzoat und/oder Ethylbenzoat, sowie
C) 5 bis 30 Gew.-% an einem oder mehreren Malonsäure-di-C₁-C₄-estern, vorzugsweise Diethylmalonat und/oder Dibutylmalonat, sowie
D) 0 bis 40 Gew.-% an gegebenenfalls einem oder mehreren Lösungsvermittlern ausgewählt aus der Gruppe bestehend aus Glycerin, Ethylenglykol und 1,2-Propylenglykol, vorzugsweise 1,2-Propylenglykol,
wobei die Gewichtsprozentangaben bezogen sind auf die Gesamtmenge der Zusammensetzung.

In den erfindungsgemäßen Zusammensetzungen tragen der oder die Benzoesäure-C₁-C₄-ester als Bestandteil B) wesentlich zur Funktion als Vergällungsmittel bei; diese Benzoesäure-C₁-C₄-ester riechen bzw. schmecken nicht angenehm. Bei Wasserzugabe zu mit einem oder mehreren Benzoesäure-C₁-C₄-estern vergälltem Ethanol wurde in eigenen Untersuchungen jedoch häufig eine unerwünschte Trübung beobachtet.

In den erfindungsgemäßen Zusammensetzungen tragen der oder die Milchsäure-C₂-C₆-ester als Bestandteil A) wesentlich zur universelleren Einarbeitbarkeit der erfindungsgemäßen Zusammensetzungen in kosmetische oder mundhygienische ethanolhaltige Produkte bei: diese Milchsäure-C₂-C₆-ester riechen bzw. schmecken leicht fruchtig und cremig-buttrig (Ethyllactat etwas fruchtig und buttrig, n-Butyllactat cremig, milchig, süßlich) und sind daher sensorisch vergleichsweise angenehm. Ferner mindern die Milchsäure-C₂-C₆-ester als Bestandteil A) der erfindungsgemäßen Zusammensetzungen die Trübungsneigung der Benzoesäure-C₁-C₄-ester als Bestandteil B) bei Zugabe von Wasser zu erfindungsgemäß vergälltem Ethanol in vorteilhafter Weise.

Der oder die Malonsäure-di-C₁-C₄-ester als Bestandteil C) erleichtern die Einarbeitung der erfindungsgemäßen Zusammensetzungen in kosmetische oder mundhygienische ethanolhaltige Produkte; die Malonsäure-di-C₁-C₄-ester, insbesondere Diethylmalonat, riechen bzw. schmecken leicht fruchtig (Diethylmalonat etwas ananasartig) und sind daher sensorisch vergleichsweise angenehm. Durch die Anwesenheit der Malonsäure-di-C₁-C₄-ester als Bestandteil C) werden erfindungsgemäße Zusammensetzungen sensorisch, insbesondere geschmacklich, unauffälliger und als weniger störend empfunden.

Der oder die optionalen Bestandteile D) Glycerin, Ethylenglykol und/oder 1,2-Propylenglykol dienen im vorliegenden Falle im Wesentlichen als Lösungsvermittler, so dass die oben beschriebenen unerwünschten Trübungen und/oder Entmischungen regelmäßig unterbleiben.

Bevorzugt sind erfindungsgemäße Zusammensetzungen umfassend oder bestehend aus:
A) Ethyllactat,
B) Ethylbenzoat,
C) Diethylmalonat, und
D) 1,2-Propylenglykol,

Generell bevorzugt ist eine erfindungsgemäße Zubereitung, bei der die Gesamtmenge der Komponenten A, B, C und D zumindest 95 Gew.-%, bevorzugt zumindest 98 Gew.-%, der Zusammensetzung ausmacht

Die bevorzugten gewichts bezogenen Mengenvemältnisse sind dabei jeweils die folgenden:
A) 40 bis 70 Gew.-% des oder der Milchsäure-C₂-C₆-ester, vorzugsweise Ethyllactat,
B) 5 bis 15 Gew.-% des oder der Benzoesäure-C₁-C₄-ester, vorzugsweise Ethylbenzoat,
C) 10 bis 25 Gew.-% des oder der Malonsäure-di-C₁-C₄-ester, vorzugsweise Diethylmalonat, und
D) 10 bis 30 Gew.-%, Glycerin, Ethylenglykol und/oder 1,2-Propylenglykol,
wobei die Gewichtsprozentangaben bezogen sind auf die Gesamtmenge der Zusammensetzung.

Die erfindungsgemäßen Zusammensetzungen (Vergältungsmittel für Ethanol) weisen lediglich einen schwachen Eigengeruch bzw. Eigengeschmack auf, der in kosmetischen und mundhygienischen Produkten als sensorisch unauffällig empfunken wird. Ferner sind die erfindungsgemäßen Zusammensetzungen als Vergällungsmittel schwer von Ethanol abtrennbar.

Die erfindungsgemäßen Zusammensetzungen lassen sich auf einfache Weise durch Mischen der einzelnen Komponenten A) und B) sowie gegebenenfalls C) und/oder D) herstellen. Für die Vergällung von Ethanol wird vorzugsweise eine erfindungsgemäße Zusammensetzung in einer Menge im Bereich von 0,01 bis 5 Gew.-%, bevorzugt im Bereich von 0,05 bis 3 Gew.-%, besonders bevorzugt im Bereich von 0,1 bis 2 Gew.-%, eingesetzt, bezogen auf das Gesamtgewicht von erfindungsgemäßer Zusammensetzung und Ethanol.

Die erfindungsgemäßen Zusammensetzungen sind in kosmetischen und mundhygienischen Produkten universell einsetzbar. Die erfindungsgemäßen Zusammensetzungen verhalten sich in erfindungsgemäßen kosmetischen und mundhygienischen Produkten (siehe dazu unten) in den allermeisten Fällen chemischphysikalisch und sensorisch nicht störend, d.h. es kommt nicht zu Wechseiwirkungen, die den jeweiligen geplanten Einsatzzweck der kosmetischen und mundhygienischen Produkte beeinträchtigen.

Die Erfindung betrifft auch eine Mischung, bestehend aus:
- Ethanol in einer Menge von 95 bis 99,95 Gew.-%,
- einer erfindungsgemäßen Zusammensetzung in einer Menge von 0,01 bis 5 Gew.-%,
bezogen auf das Gesamtgewicht der erfindungsgemäßen Zusammensetzung und Ethanol,
sowie gegebenenfalls weiteren Inhaltsstoffen.

Bei Zugabe von Wasser zu einer erfindungsgemäßen Mischung, d. h. erfindungsgemäß vergälltem Ethanol bzw. beim Vermischen von Wasser und erfindungsgemäß vergälltem Ethanol kommt es nicht zu Trübungen oder Entmischung der Phasen, insbesondere nicht zur Ausbildung eines Ölfilms oder von "Fettaugen".

Eine bevorzugte erfindungsgemäße Mischung umfasst:
- Ethanol in einer Menge von 97 bis 99,95 Gew.-%, bevorzugt 98 bis 99,9 Gew.-%, und
- eine erfindungsgemäße Zusammensetzung in einer Menge von 0 0,05 bis 3 Gew.-%, bevorzugt 0,1 bis 2 Gew.-%,
bezogen auf das Gesamtgewicht der Zusammensetzung und Ethanol,

Eine besonders bevorzugte erfindungsgemäße Mischung (vergälltes Ethanol) umfasst:
70 bis 99,9 Gew.-% Ethanol,
bezogen auf das Gesamtgewicht der Mischung.

Vorzugsweise ist eine erfindungsgemäße Zusammensetzung frei von Diethylphthalat.

Ein weiterer Gegenstand der Erfindung sind mit der erfindungsgemäßen Zusammensetzung bzw, Mischung hergestellte kosmetische oder mundhygienische Produkte. Diese Produkte können 1 bis 99 Gew.-%, bezogen auf ethanol-haltiges Produkt, der erfindungsgemäßen Zusammensetzung enthalten.

Derartige kosmetische Produkte umfassen (bevorzugter Ethanolgehalt in Klammem) Extrait, Eau de Parfum, Eau de Toilette, Eau de Cologne (jeweils 30 - 99 Gew.-%), Splash Cologne, Rasierwasser, allgemeine Dea-Formuiiemngen, Haarfestiger, Haarspray, Haargele (1-50 Gew.-%), Gesichtswasser (5-70 Gew.-%), Körpergele, in besonderen Emulsionen (jeweils 1-40 Gew.-%), Haarwasser (10-75 Gew.-%), Handdesinfektionsmittel (20-99 Gew.-%). Glasreiniger (10-90 Gew.-%), Erfrischungstücher (30-90 Gew.-%) usw.

Bevorzugte ethanolhaltige mundhygienische Produkte (ethanolhaltige Mundpflegeprodukte) sind solche in Form von Zahnpasta, Zahncreme, Zahngel, Zahnpulver, Zahnputzflüssigkeit, Zahnputzschaum, Mundwasser, Mundwasserkonzentrat, Zahncreme und Mundwasser als 2-in-1 Produkt, Lutschbonbon, Mundspray, Zahnseide, oder Zahnpflegekaugummi. Am meisten bevorzugt sind Mundwasser, Mundwasserkonzentrat, Zahncreme und Mundwasser als 2-in-1 Produkt.

Weitere Aspekte der vorliegenden Erfindung ergeben sich aus den beigefügten Patentansprüchen und den nachfolgenden Beispielen.

Die Teile und Prozentangaben der nachfolgenden Beispiele beziehen sich jeweils auf das Gewicht.

### Beispiele

### Beispiel 1: Erfindungsgemäße Zusammensetzungen

| **Bestandteil** | | **Z1** | **Z2** | **Z3** |
|---|---|---|---|---|
| | | Gewichtsteile | Gewichtsteile | Gewichtsteile |
| | | | | |
| A | Ethyllactat | 640 | 560 | 530 |
| B | Ethylbenzoat | 150 | 80 | 100 |
| C | Diethylmalonat | 60 | 160 | 200 |
| C | Di-n-butylmalonat | 90 | - | - |
| D | Ethylenglykol | - | - | 50 |
| D | 1,2-Propylenglykol | 60 | 200 | 120 |
| Total: | | 1000 | 1000 | 1000 |

### Beispiel 2: Aromastoffkomposition (synthetisches Pfefferminzöl), nicht erfindungsgemäß

| | **Aroma A1** |
|---|---|
| | Gewichtsteile |
| | |
| Isobutyraldehyd | 0,5 |
| 3-Octanol | 0,5 |
| Dimethylsulfid | 0,5 |
| Trans-2-Hexenal | 1,0 |
| Cis-3-Hexenol | 1,0 |
| 4-Terpineol, natürlich | 1,0 |
| Isopulegol | 1,0 |
| Piperiton, natürlich, aus Eucalyptus | 2,0 |
| Linalool | 3,0 |
| 8-Ocimenylactetat, 10% in Triacetin | 5,0 |
| Isoamylalkohol | 10,0 |
| Isovaleraldehyd | 10,0 |
| Alpha-Pinen | 25,0 |
| Beta-Pinen, natürlich | 25,0 |
| Neomenthol, racemisch | 40,0 |
| Eucalyptol (1,8-Cineol), natürlich | 50,0 |
| L-Menthylacetat | 70,0 |
| L-Menthon | 220,0 |
| D-Isomenthon | 50,0 |
| L-Menthol | 484,5 |
| Total: | 1000,0 |

Aroma A1 wird in Produkten eingesetzt, die in den nachfolgenden Beispielen F1 bis F5 definiert sind.

### Beispiel F1: Gebrauchsfertiges Mundwasser mit Fluorid

| | I (Gew.-%) | II (Gew.-%) | III (Gew.-%) |
|---|---|---|---|
| Ethanol | 7,00 | 7,00 | 7,00 |
| Mischung Z2 aus Beispiel 1 | 0,05 | 0,07 | 0,10 |
| Glycerin | 12,00 | 12,00 | 12,00 |
| Na-fluorid | 0,05 | 0,05 | 0,05 |
| Pluronic F-127^{®} (BASF, oberflächenaktive Substanz) | 1,40 | 1,40 | 1,40 |
| Na-phosphatpuffer pH 7,0 | 1,10 | 1,10 | 1,10 |
| Sorbinsäure | 0,20 | 0,20 | 0,20 |
| Na-saccharinat | 0,10 | 0,10 | 0,10 |
| Aroma A1 aus Beispiel 2 | 0,25 | 0,25 | 0,40 |
| Blauer Farbstoff | 0,01 | 0,01 | 0,01 |
| Wasser dest. | Ad 100,00 | Ad 100,00 | Ad 100,00 |

### Beispiel F2: Mundwasserkonzentrat

| | I (Gew.-%) | II (Gew.-%) | III (Gew.-%) |
|---|---|---|---|
| Ethanol, 96%ig | 80,00 | 80,00 | 80,00 |
| Mischung Z1 aus Beispiel 1 | 0,50 | 0,80 | 1,10 |
| Na-cyclamat | 0,15 | 0,15 | 0,15 |
| Spearmint-Aroma (enthält natürliches I-Carvon) | 1,00 | 0,50 | 1,50 |
| Farbstoff | 0,01 | 0,01 | 0,01 |
| Aroma A1 aus Beispiel 2 | 2,00 | 3,00 | 2,50 |
| Wasser dest. | Ad 100,00 | Ad 100,00 | Ad 100,00 |

### Beispiel F3: Mundwasserkonzentrat mit Zinkchlorid und Cetylpyridiniumchlorid

| | I (Gew.-%) | II (Gew.-%) | III (Gew.-%) |
|---|---|---|---|
| Ethanol | 42,00 | 42,00 | 42,00 |
| Mischung Z2 aus Beispiel 1 | 0,25 | 0,50 | 0,75 |
| Cremophor RH 455 | 5,00 | 5,00 | 5,00 |
| Zinkchlorid | 0,50 | 0,50 | 0,50 |
| Cetylpyridiniumchlorid | 0,50 | 0,50 | 0,50 |
| Na-saccharinat | 0,10 | 0,10 | 0,10 |
| Roter Farbstoff | 0,01 | 0,01 | 0,01 |
| Aroma A1 aus Beispiel 2 | 2,00 | 2,50 | 3,00 |
| Wasser dest. | Ad 100,00 | Ad 100,00 | Ad 100,00 |

### Beispiel F4: Transparentes Zahngel mit Alkohol

| | I (Gew.-%) |
|---|---|
| Ethanol, 96%ig | 1,40 |
| Mischung Z2 aus Beispiel 1 | 0,01 |
| Sorbitol, 70 %ig in Wasser | 61,50 |
| Saccharin | 0,20 |
| Na-Monofluorphosphat | 0,80 |
| Polyethylenglykol PEG 32 (Polylglycol 1500 S) | 2,00 |
| Abrasivkieselsäure (Sident 9) | 10,00 |
| Verdickungskieselsäure (Sident 22S) | 8,00 |
| Na-Carboxymethylcellulose (Walcocel 2000 PPA 12) | 0,50 |
| Natriumlaurylsulfat | 1,46 |
| Roter und blauer Farbstoff | 0,01 |
| Aroma A1 aus Beispiel 2 | 1,00 |
| Wasser dest. | Ad 100,00 |

### Beispiel F5: Zahncreme und Mundwasser als 2-in-1

| | I (Gew.-%) |
|---|---|
| Ethanol, 96%ig | 5,00 |
| Mischung Z2 aus Beispiel 1 | 0,04 |
| Sorbitol, 70 %ig in Wasser | 40,00 |
| Glycerin | 20,00 |
| Saccharin | 0,20 |
| Na-Monofluorphosphat | 0,76 |
| Solbrol M, Na-Salz | 0,15 |
| Abrasivkieselsäure (Sident 9) | 20,00 |
| Verdickungskieselsäure (Sident 22S) | 2,00 |
| Na-Carboxymethylcellulose | 0,30 |
| Natriumlaurylsulfat | 1,20 |
| grüner Farbstoff (1%ig in Wasser) | 0,50 |
| Aroma A1 aus Beispiel 2 | 1,00 |
| Wasser dest. | Ad 100,00 |

### Beispiel F6: Aerosol-Pflegehaarspray

| | I (Gew.-%) |
|---|---|
| Ethanol | 40,00 |
| Mischung Z3 aus Beispiel 1 | 0,20 |
| Sulfonierte Polyester (z.B. Kammpolymere gemäß DE 199 09 757) | 8,00 |
| Dimethylether | 40,00 |
| Parfüm | 0,15 |
| Vitamin-E-acetat und Konservierungsmittel | 0,10 |
| UV-Filter | 0,10 |
| Wasser dest. | Ad 100,00 |

### Beispiel F7: Nonaerosol-Haarspray mit extra starker Festigung

| | I (Gew.-%) |
|---|---|
| Ethanol | 55,00 |
| Mischung Z2 aus Beispiel 1 | 0,42 |
| Sulfonierte Polyester (z.B. Kammpolymere gemäß DE 199 09 757) | 10,00 |
| Parfüm | 0,15 |
| Vitamin-E-acetat und Konservierungsmittel | 0,10 |
| UV-Filter | 0,10 |
| Wasser dest. | Ad 100,00 |

## Patentansprüche

1. Zusammensetzung umfassend oder bestehend aus;
A) 20 bis 80 Gew.-% an einem oder mehreren Milchsäure-C₂-C₆-estern, vorzugsweise Ethyllactat, n-Butyllactat, iso-Amyllactat und/oder n-Amyllactat, und
B) 1 bis 20 Gew.-% an einem oder mehreren Benzoesäure-C₁-C₄-estern, vorzugsweise Methylbenzoat und/oder Ethylbenzoat, sowie
C) 5 bis 30 Gew.-% an einem oder mehreren Malonsäute-di-C₁-C₄-estern, vorzugsweise Diethylmalonat und/oder Dibutylmalonat, sowie
D) 0 bis 40 Gew.-% an einem oder mehreren Lösungsvermittlern ausgewählt aus der Gruppe bestehend aus Glycerin, Ethylenglykol und 1,2-Propylenglykol, vorzugsweise 1,2-Propylenglykol,
wobei die Gewichtsprozentangaben bezogen sind auf die Gesamtmenge der Zusammensetzung.

2. Zusammensetzung nach Anspruch 1, wobei der eine bzw, einer der mehreren Milchsäure-C₂-C₆-ester Ethyllactat ist.

3. Zusammensetzung nach einem der vorangehenden Ansprüche, wobei der eine bzw. einer der mehreren Benzoesäure-C₁-C₄-ester Ethylbenzoat ist.

4. Zusammensetzung nach einem der vorangehenden Ansprüche, wobei die Zusammensetzung ein oder mehrere Malonsäure-di-C₁-C₄-estern umfasst und der eine bzw. einer der mehreren Malonsäure-di-C₁-C₄-ester Dimethylmalonat ist.

5. Zusammensetzung nach einem der vorangehenden Ansprüche, wobei die Zusammensetzung 1,2-Propylenglykol umfasst.

6. Zusammensetzung nach einem der vorangehenden Ansprüche, umfassend oder bestehend aus;
A) Ethyllactat sowie gegebenenfalls einem oder mehreren weiteren Milchsäure-C₂-C₆-estern,
B) Ethylbenzoat sowie gegebenenfalls einem oder mehreren weiteren Benzoesäure-C₁-C₄-ester,
C) Diethylmalonat sowie gegebenenfalls einem oder mehreren weiteren Malonsäure-di-C₁-C₄-ester, und
D) 1,2-Propylenglykol sowie gegebenenfalls Glycerin und/oder Ethylenglykol.

7. Zusammensetzung nach einem der vorangehenden Ansprüche, umfassend oder bestehend aus:
A) Ethyllactat,
B) Ethylbenzoat,
C) Diethylmalonat, und
D) 1,2-Propylenglykol.

8. Zusammensetzung nach einem der vorangehenden Ansprüche, wobei die Gesamtmenge der Komponenten A, B, C und D zumindest 95 Gew.-%, bevorzugt zumindest 98 Gew.-%, der Zusammensetzung ausmacht.

9. Zusammensetzung nach einem der vorangehenden Ansprüche, umfassend die folgenden Mengen der Komponenten A, B, C und D:
A) 40 bis 70 Gew..-% des oder der Milchsäure-C₂-C₆-ester,
B) 5 bis 15 Gew.-% des oder der Benzoesäure-C₁-C₄-ester,
C) 10 bis 25 Gew.-% des oder der Malonsäure-di-C₁-C4-ester, und
D) 10 bis 30 Gew.-% des oder der Lösungsvermittler,
wobei die Gewichtsprozentangaben bezogen sind auf die Gesamtmenge der Zusammensetzung.

10. Mischung, bestehend aus:
- Ethanol in einer Menge von 95 bis 99,95 Gew.-%,
- einer Zusammensetzung nach einem der Ansprüche 1 bis 9 in einer Menge von 0.01 bis 5 Gew.-%.
bezogen auf das Gesamtgewicht der Zusammensetzung nach einem der Ansprüche 1 bis 9 und Ethanol,
sowie gegebenenfalls
- weiteren Inhaltsstoffen.

11. Mischung nach Anspruch 10, umfassend
- Ethanol in einer Menge von 97 bis 99,95 Gew.-%, bevorzugt 98 bis 99,9 Gew.-%, und
- die Zusammensetzung nach einem der Ansprüche 1 bis 9 in einer Menge von 0,05 bis 3 Gew.-%, bevorzugt 0,1 bis 2 Gew.-%,
bezogen auf das Gesamtgewicht der Zusammensetzung nach einem der Ansprüche 1 bis 9 und Ethanol.

12. Mischung nach Anspruch 10 oder 11, umfassend 70 bis 99,9 Gew.-% Ethanol, bezogen auf das Gesamtgewicht der Mischung.

13. Kosmetisches oder mundhygienisches Produkt, umfassend eine Mischung nach einem der Ansprüche 10 bis 12, wobei das Produkt vorliegt als:
Extrait, Eau de Parfum, Eau de Toilette, Eau de Cologne, Splash Cologne, Rasierwasser, Deo-Formulierung, Haarfestiger, Haarspray, Haargele, Gesichtswasser, Körpergel, Haarwasser, Handdesinfektionsmittel, Glasreiniger, Erfrischungstuch, Zahnpasta, Zahncreme, Zahngel, Zahnpulver, Zahnputzflüssigkeit, Zahnputzschaum, Mundwasser, Mundwasserkonzentrat, Zahncreme und Mundwasser als 2-in-1 Produkt, Lutschbonbon, Mundspray, Zahnseide, Zahnpflegekaugummi.

14. Verwendung einer Zusammensetzung nach einem der Ansprüche 1 bis 9 als Vergällungsmittel für Ethanol.

15. Verwendung einer Mischung nach einem der Ansprüche 10 bis 12 zur Herstellung eines kosmetischen oder mundhygienischen Produktes.

## Claims

1. Composition comprising or consisting of:
A) 20 to 80 wt.-% of one or more lactic acid-C₂-C₆-esters, preferably ethyl lactate, n-butyl lactate, iso-amyl lactate and/or n-amyl lactate, and
B) 1 to 20 wt.-% of one or more benzoic acid-C₁-C₄-esters, preferably methyl benzoate and/or ethyl benzoate, as well as
C) 5 to 30 wt.-% of one or more malonic acid-di-C₁-C₄-esters, preferably diethyl malonate and/or dibutyl malonate, as well as
D) 0 to 40 wt.-% of one or more solubilizers chosen from the group consisting of glycerine, ethylene glycol and 1,2-propylene glycol, preferably 1,2-propylene glycol
wherein the wt.-% values are based on the total amount of the composition.

2. Composition according to claim 1, wherein the one or, respectively, one of the lactic acid-C₂-C₆-esters is ethyl lactate.

3. Composition according to any of the preceding claims, wherein the one or, respectively, one of the benzoic acid-C₁-C₄-esters is ethyl benzoate.

4. Composition according to any of the preceding claims, wherein the composition comprises one or more malonic acid-di-C₁-C₄-esters and the one or respectively one of the malonic acid-di-C₁-C₄-esters is diethyl malonate.

5. Composition according to any of the preceding claims, wherein the composition comprises 1,2-propylene glycol.

6. Composition according to any of the preceding claims, comprising or consisting of:
A) ethyl lactate as well as where appropriate one or more additional lactic acid-C₂-C₆-esters.
B) ethyl benzoate as well as where appropriate one or more additional benzoic acid-C₁-C₄-esters,
C) diethyl malonate as well as where appropriate one or more additional malonic acid-di-C₁-C₄-esters, and
D) 1,2-propylene glycol as well as where appropriate glycerine and/or ethylene glycol.

7. Composition according to any of the preceding claims, comprising or consisting of
A) ethyl lactate,
B) ethyl benzoate,
C) diethyl malonate, and
D) 1,2-propylene glycol

8. Composition according to any of the preceding claims, wherein the total amount of the components A, B, C and D amounts to at least 95 wt.-%, preferably at least 98 wt.-%.

9. Composition according to any of the preceding claims, comprising the following amounts of the components A, B, C and D:
A) 40 to 70 wt.-% of said lactic acid-C₂-C₆-ester(s),
B) 5 to 15 wt.-% of said benzoic acid-C₁-C₄-ester(s),
C) 10 to 25 wt.-% of said malonic acid-di-C₁-C₄-ester(s), and
D) 10 to 30 wt.-% of said solubilizer(s).

10. A mixture consisting of:
- ethanol in an amount of 95 to 99,95 wt.-%
- a composition according to any of the preceding claims in an amount of 0,01 to 5 wt.%,
based on the total weight of the composition according to any of claims 1 to 9 and ethanol,
- as well as, where appropriate, additional ingredients.

11. A mixture according to claim 10,comprising:
- ethanol in the amount of 97 to 99.95 wt.-%, preferably 98 to 99.9 wt.-%,and
- the composition according to any of claims 1 to 9 in an amount of 0.05 to 3 wt.-% , preferably 0.1 to 2 wt.-%
based on the total weight of the composition according to any of claims 1 to 9 and the ethanol.

12. A mixture according to claim 10 or 11, comprising 70 to 99.9 wt.-% of ethanol, based on the total weight of the mixture

13. Cosmetic or oral hygienic product, composed of a mixture according to any of claims 10-12, where the product is available as :
extrait, eau de parfum, eau de toilette, eau de Cologne, splash Cologne, aftershave, deodorant, hair mouse, hairspray, hair gel, face tonic, body gel, hair tonic, hand disinfectant, window cleaner, refreshment cloths, toothpaste, tooth powder, tooth cleaning liquid, tooth cleaning foam, mouthwash, mouthwash concentrate, toothpaste and mouthwash as a 2-in-1 product, boiled sweets, mouth spray, dental floss, dental chewing gum.

14. Usage of a composition according to any of claims 1-9 as a denaturant for ethanol.

15. Usage of a mixture according to any of claims 10-12 to produce a cosmetic or an oral hygienic product.

## Revendications

1. Composition comprenant ou constituée :
A) de 20 à 80 % en poids d'un ou de plusieurs esters C₂-C₆ d'acide lactique, préférentiellement de lactate d'éthyle, de lactate de n-butyle, de lactate d'isoamyle et/ou de lactate de n-amyle, et
B) de 1 à 20 % en poids d'un ou de plusieurs esters C₁-C₄ d'acide benzoïque, préférentiellement de benzoate de méthyle et/ou de benzoate d'éthyle, ainsi que
C) de 5 à 30 % en poids d'un ou de plusieurs di-esters C₁-C₄ d'acide malonique, préférentiellement de malonate de diéthyle et/ou de malonate de dibutyle, ainsi que
D) de 0 à 40 % en poids d'un ou de plusieurs agents de solubilisation choisis dans le groupe constitué par le glycérol, l'éthylèneglycol et le 1,2-propylène glycol, s'agissant préférentiellement de 1.2-propylène glycol,
les pourcentages en poids se rapportant à la quantité totale de la composition.

2. Composition selon la revendication 1, ledit ou, respectivement, l'un desdits plusieurs esters C₂-C₆ étant le lactate d'éthyle.

3. Composition selon l'une des revendications précédentes, ledit ou, respectivement, l'un desdits plusieurs esters C₁-C₄ d'acide benzoïque étant le benzoate d'éthyle.

4. Composition selon l'une des revendications précédentes, ladite composition comprenant un ou plusieurs di-esters C₁-C₄ d'acide malonique, et ledit ou, respectivement, l'un desdits plusieurs di-esters C₁-C₄ d'acide malonique étant le malonate de diéthyle.

5. Composition selon l'une des revendications précédentes, ladite composition comprenant le 1,2-propylène glycol.

6. Composition selon l'une des revendications précédentes, comprenant ou constituée :
A) de lactate d'éthyle ainsi que, le cas échéant, d'un ou de plusieurs autres esters C₂-C₆ d'acide lactique,
B) de benzoate d'éthyle ainsi que, le cas échéant, d'un ou de plusieurs autres esters C₁-C₄ d'acide benzoïque,
C) de malonate de diéthyle ainsi que, le cas échéant, d'un ou de plusieurs autres di-esters C₁-C₄ d'acide malonique, et
D) de 1,2-propylène glycol ainsi que, le cas échéant, de glycérol et/ou de l'éthylèneglycol.

7. Composition selon l'une des revendications précédentes, comprenant ou constituée :
A) de lactate d'éthyle,
B) de benzoate d'éthyle.
C) de malonate de diéthyle et
D) de 1,2-propylène glycol.

8. Composition selon l'une des revendications précédentes, la quantité totale des composants A, B, C et D constituant au moins 95 % en poids, de préférence au moins 98 % en poids, de ladite composition.

9. Composition selon l'une des revendications précédentes, comprenant les quantités suivantes des composants A, B. C et D :
A) 40 à 70 % en poids dudit ou desdits esters C₂-C₆ d'acide lactique,
B) 5 à 15 % en poids dudit ou desdits esters C₁-C₄ d'acide benzoïque.
C) 10 à 25 % en poids dudit ou desdits di-esters C₁-C₄ d'acide malonique, et
D) 10 à 30 % en poids dudit ou desdits agents de solubilisation,
les pourcentages en poids se rapportant à la quantité totale de la composition.

10. Mélange, constitué :
- d'éthanol dans une quantité comprise entre 95 et 99,95 % en poids,
- d'une composition selon l'une des revendications 1 à 9 dans une quantité comprise entre 0,01 et 5 % en poids,
par rapport au poids total de ladite composition selon l'une des revendications 1 à 9 et l'éthanol,
ainsi que, le cas échéant.
- d'autres ingrédients.

11. Mélange selon la revendication 10, comprenant
- de l'éthanol dans une quantité comprise entre 97 et 99,95 % en poids, de préférence entre 98 et 99,9 % en poids, et
- ladite composition selon l'une des revendications 1 à 9 dans une quantité comprise entre 0,05 et 3 % en poids, de préférence entre 0,1 et 2 % en poids,
par rapport au poids total de ladite composition selon l'une des revendications 1 à 9 et l'éthanol.

12. Mélange selon les revendications 10 ou 11, comprenant 70 à 99,9 % en poids d'éthanol, par rapport au poids total dudit mélange.

13. Produit cosmétique ou d'hygiène buccale, comprenant un mélange selon l'une des revendications 10 à 12, ledit produit se présentant sous forme :
d'un extrait, d'une eau de parfum, d'une eau de toilette, d'une eau de Cologne, d'une eau de Cologne fraîche, d'une eau après-rasage, d'une formulation pour déodorants, d'un produit de fixation de coiffure, d'une laque de coiffure, de gels de coiffure, d'une eau pour le visage, d'un gel pour le corps, d'une lotion capillaire, d'un désinfectant pour les mains, d'un nettoyant pour surfaces vitrées, d'une lingette rafraîchissante, d'un dentifrice, d'une crème dentifrice, d'un gel dentifrice, d'une poudre dentifrice, d'un liquide dentifrice, d'une mousse dentifrice, d'un bain de bouche, d'un bain de bouche concentré, d'une crème dentifrice et bain de bouche 2-en-1, d'un bonbon à sucer, d'un spray buccal, d'un fil dentaire, d'un chewing-gum dentifrice.

14. Utilisation d'une composition selon l'une des revendications 1 à 9 en tant qu'agent de dénaturation pour l'éthanol.

15. Utilisation d'un mélange selon l'une des revendications 10 à 12 pour la préparation d'un produit cosmétique ou d'hygiène buccale.
